# EUROPEAN PATENT APPLICATION

(11) **EP 0 870 474 A1**
(43) Date of publication of application: **14.10.1998**
(21) Application number: 97932846.5
(22) Date of filing: 28.07.1997
(51) Int. Cl.: A61B 17/70, F16B 35/00

(54) **IMPROVEMENTS TO DEVICES FOR FIXING AND CORRECTING DORSAL VERTEBRAE**

(30) Priority: 07.08.1996 ES 9600176
(71) Applicant: Bioprot, S.A., 43005 Tarragona (ES)
(72) Inventor: VAZQUEZ SOLSONA, D., Juan José, E-43005 Tarragona (ES)
(74) Representative: Duran Moya, Luis-Alfonso
(86) International application number: ES9700191
(87) International publication number: WO9805262

(57) **Abstract**

The improvements are applicable to devices for fixing and correcting dorsal vertebrae, of the type which comprise a screw head provided with a groove for receiving a rod for the correction and/or fixing of vertebrae, which is fixed by a nut threaded on the screw head; the nut has at its lower edge an asymmetric toothing with attack and drive edges in the proper rod to be fixed, thereby providing for its rapid and efficient fixing as well as for the braking of the upper nut itself. The head of this screw has a reduced and externally threaded apart to receive the fixing nut, the external diameter of the nut not exceeding the external diameter of the enlarged part.

## Description

The present invention relates to some improvements introduced in elements for fixing and correcting dorsal vertebrae, bringing considerable improvements by reason of their greater simplicity and effectiveness.

The improvements of the present invention relate to screws for fixing in regions of the vertebral column for procedures to fix and/or correct traumatic, degenerative and tumoral processes.

Special screws for this function are already known from Spanish Patent application No. 9101092 which disclosed a screw with a recess in the head in which a cap was inserted, the cap bearing the pressure of an external nut which was coupled with a threaded region of the head of the screw, exerting pressure on the bar of the correcting device by means of the element or cap housed therein.

The improvements of the present invention are intended to make known an improved screw and nut assembly for securing the correcting and fixing rods which are generally used in the thoracic-lumbar and lumbar-sacral regions of the vertebral column.

The object of the present improvements is to simplify special screw and nut assemblies for this securing function, achieving effective operation with a minimal number of elements and at the same time achieving effective immobilization of the device for gripping the rod.

Basically, the present improvements are applied to a screw with a head having a recess for the insertion of the rod with the innovation that, instead of acting on the periphery of the rod by means of a cap, the outer fixing nut acts thereon directly by means of a set of teeth of the lower peripheral face of the nut which acts directly on the rod, simultaneously compressing and immobilizing the rod. This simple arrangement can be put into effect in a simplified manner and permits quick and effective placing of the screws, as well as a secure grip of the nuts for immobilizing the rods.

The set of teeth which is provided on the lower peripheral rim of the nut of this device has a plurality of individual teeth shaped as circular sectors, extending over the whole of the said peripheral rim and having a slight inclination, preferably of about five degrees. The shape of the individual teeth corresponds substantially to a retaining ring in which the tooth profile adopts approximately the shape of a right-angled triangle. The edge of the tooth bites into the rod of the device, achieving rapid immobilization which considerably facilitates the surgeon's task.

A further improvement provided by the present invention is that the head of the screw which receives the rod is formed with a recessed structure in its upper portion, that is, the portion which is situated above the region receiving the rod since, for a given diameter of rod and without reducing the thickness of the lateral walls of its nut in the strong portion thereof, a narrower portion is thus obtained which, once its lower surface has been threaded, permits the coupling of a nut, the external diameter of which can correspond to that of the head of the screw so that there are practically no projecting regions in the assembly incorporating the screw and its nut.

For a better understanding, some drawings of a preferred embodiment of the improvements of the present invention are appended by way of non-limiting example.

Figure 1 shows a transverse section, taken in the plane indicated, of a fixing screw which incorporates the improvements of the invention.

Figure 2 is a longitudinally-sectioned elevational view of an entire screw head formed in accordance with the improvements of the invention.

Figure 3 shows a plan view of the nut for fixing on the screw, showing the toothed region.

Figures 4 and 5 are a side elevational view and a plan view of the nut, respectively.

Figure 6 shows a transverse section of the nut itself, taken in the section plane indicated.

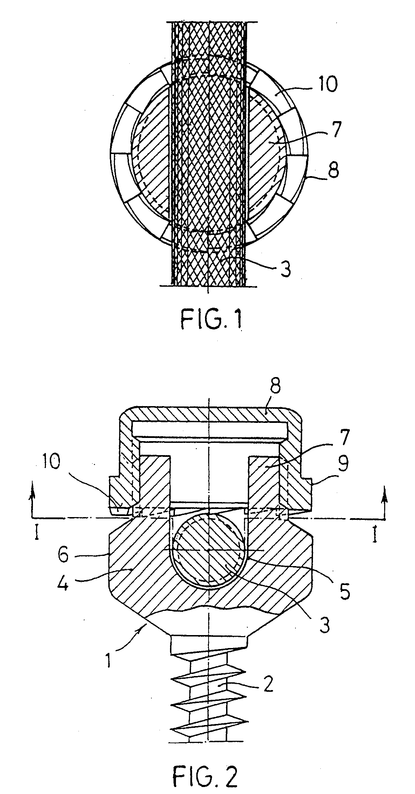

As will be appreciated from the drawings, the improvements of the present invention provide for the head 1 of a screw 2 which is intended to secure rods 3 for fixing and/or correcting regions of the vertebral column to be formed by a lower portion 4 having an internal groove 5 for receiving the rod 3, and an outer periphery 6 the diameter of which is determined by the requirements for the strength of the screw itself. The region 4 of the head 1 is extended at the top by an externally-recessed, cylindrical region 7 having, on its outer surface, a screw thread which can be seen coupled with the upper nut 8 in Figure 2.

The nut 8 has a maximum diameter corresponding, in the embodiment shown, to the annular or thickened base 9 of the nut, which preferably does not exceed the diameter of the wider portion 4 of the head of the screw so that there are no projections in the head of the screw once the nut is fitted.

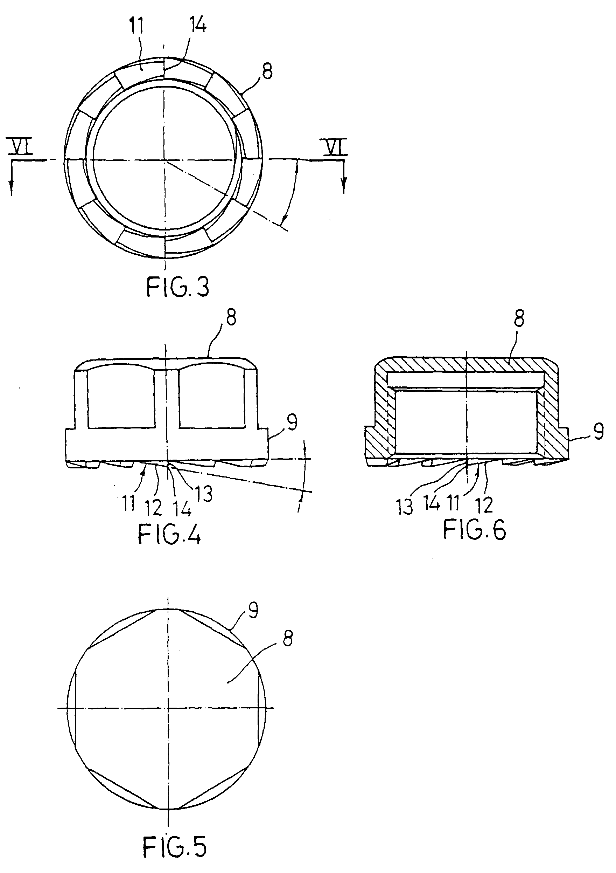

For securing the nut quickly and easily on the rod 3, the nut has, in its lower edge, a set of asymmetric teeth 10 preferably with a right-angled triangular tooth profile, as can be appreciated from Figure 4, in which it is possible to see one of the teeth 11 having a gently inclined side 12 and a straight side 13 defining a tip or vertex 14 which can bite into the rod 3, as can be appreciated from Figure 2. When the nut 8 is fitted on the cylindrical threaded neck 7, the rod is thus immobilized and secured and the nut itself is self-locked by the mechanical pressure exerted.

For the structure mentioned, it is advantageous for the lower edge 9 of the nut to have the annular or thickened shape mentioned to allow the lower surface in which the teeth 10 are disposed to have a larger area so that the teeth can have the dimensions necessary to optimize their operation.

As will be understood, the construction of the screws for securing rods for correcting and/or fixing vertebrae which is the subject of the present invention provides means for tightening the screws and securing the rods very quickly and easily with effective locking of the nuts themselves, at the same time reducing edges and external projections of the nut and screw assembly.

## Claims

1. Improvements to devices for fixing and correcting dorsal vertebrae, of the type comprising a screw head having a groove for receiving a rod for correcting and/or fixing vertebrae, the rod being fixed in the head of the screw by means of a threaded nut, characterized in that the nut has, on its lower rim, a set of asymmetric teeth with leading edges which bite into the rod itself to be fixed, permitting quick and effective securing thereof as well as locking of the nut itself.

2. Improvements to devices for fixing and correcting dorsal vertebrae according to Claim 1, characterized in that the lower rim of the nut is in the form of a ring or thickened portion in order to achieve a larger area on the lower rim carrying the set of teeth.

3. Improvements to devices for fixing and correcting dorsal vertebrae according to Claim 1, characterized in that the upper portion of the head of the screw is recessed and externally threaded in order to receive the securing nut, enabling the external diameter of the nut to be no greater than that corresponding to the wider portion of the screw, avoiding projections in the nut and screw assembly.
